(19) Europäisches Patentamt
European Patent Office
Office européen des brevets

(11) Publication number: **0 144 845**
**B1**

(12) # EUROPEAN PATENT SPECIFICATION

(45) Date of publication of patent specification: **09.03.88**

(51) Int. Cl.$^4$: **C 07 D 209/26,** A 61 K 31/405

(21) Application number: **84113917.3**

(22) Date of filing: **16.11.84**

(54) **Indolylacetic acid derivatives and preparations containing same for medical treatments.**

(30) Priority: **18.11.83 JP 218431/83**

(43) Date of publication of application:
**19.06.85 Bulletin 85/25**

(45) Publication of the grant of the patent:
**09.03.88 Bulletin 88/10**

(84) Designated Contracting States:
**BE DE FR GB NL SE**

(56) References cited:
**AT-B- 324 322**
**AT-B- 329 552**

(73) Proprietor: **THE GREEN CROSS CORPORATION**
**15-1, Imabashi-1-chome Higashi-ku Osaka-shi**
**Osaka 541 (JP)**

(72) Inventor: **Fukaya, Chikara**
**2-11-33-604, Kema-cho Miyakojima-ku**
**Osaka-shi Osaka (JP)**
Inventor: **Iwai, Masakazu**
**5-5-18, Koyama**
**Fujiidera-shi Osaka (JP)**
Inventor: **Yokoyama, Kazumasa**
**Sun-Heights 2-201, 2-7, Terauchi**
**Toyonaka-shi Osaka (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 144 845 B1

Courier Press, Leamington Spa, England.

**Description**

The present invention relates to novel indolylacetic acid derivatives and preparations containing the derivatives for medical treatment.

Indolylacetic acid-based antiphlogistics or analgesics have heretofore been prepared in the form of drugs to be administered orally or as a suppository containing indolylacetic acid as a free carboxylic acid and these preparations have been widely used clinically.

However, they are disadvantageous since they have a side effect, i.e., a serious action on gastrointestinal tracts. Thus, various proposals have heretofore been made to reduce such side effect using non-lipid substances as described in, for example, U.S. Patents 4,332,795, 4,369,182 and 4,378,354. However, satisfactory results have not been obtained heretofore with non-lipid substances. In AT—B—329 552 indolylacetic acid derivatives are described having the formula

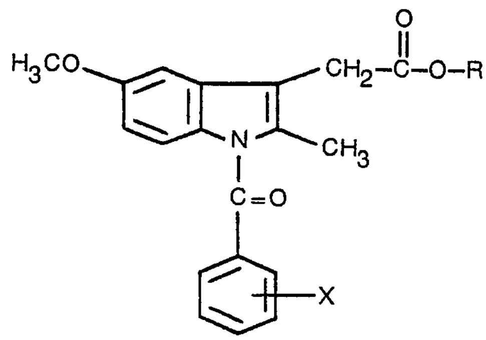

wherein R represents a benzyl group or a group of the formula

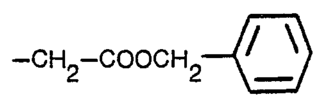 ,

wherein X represents hydrogen or one or more hydrogen atoms and/or methoxy-, nitro- or trifluormethyl groups.

Furthermore, AT—B—324 322 describes indolylacetic acid derivatives having the formula

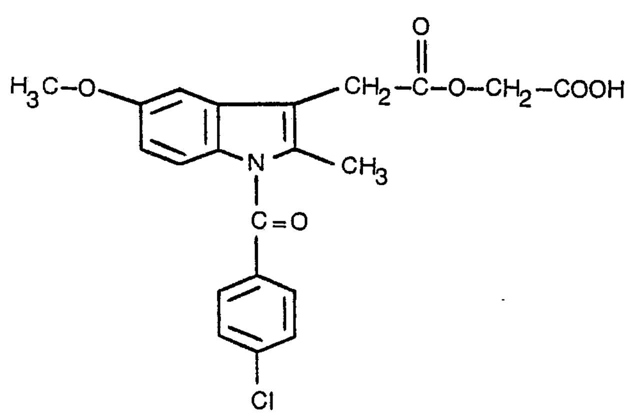

Object of the present invention is to provide novel indolylacetic acid derivatives having a high solubility in a lipid.

A further object of the present invention is to provide a preparation containing the novel indolylacetic acid derivatives which can be administered intravenously or orally.

As a result of extensive investigations, it has now been found that certain indolylacetic acid derivatives are highly soluble in a lipid and further have superior antiphlogistic activity.

That is, the present invention provides an indolylacetic acid derivative represented by the general formula (I):

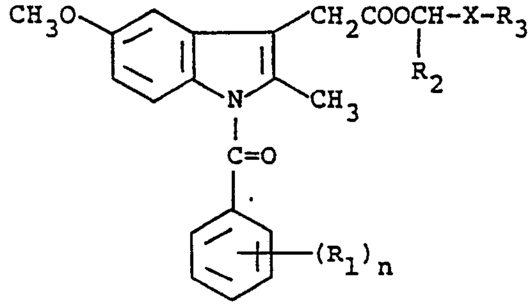

(I)

wherein $R_1$ represents a halogen atom, $R_2$ represents a hydrogen atom or an alkyl group, having from 1 to 5 carbon atoms, X represents

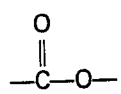 or 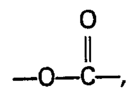,

$R_3$ represents an alkyl group having 1 to 15 carbon atoms or an alkenyl group having from 2 to 15 carbon atoms, and n is an integer of from 1 to 3.

Further, the present invention provides a preparation for medical treatment containing the indolylacetic acid derivative of (I) above as an active ingredient.

In general formula (I), $R_1$ represents a halogen atom such as a fluorine atom, a chlorine atom, a bromine atom or an iodine atom and preferably a fluorine atom. n is an integer of from 1 to 3. The indolylacetic acid derivatives of general formula (I) include dihalogeno- and trihalogeno-compounds, preferably difluoro- and trifluoro-compounds.

$R_2$ represents a hydrogen atom or an alkyl group having from 1 to 5 carbon atoms. Preferably $R_2$ represents a hydrogen atom.

X represents

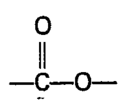 or 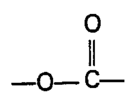

and preferably

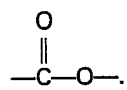.

$R_3$ represents an alkyl group having from 1 to 15 carbon atoms, preferably from 4 to 10 carbon atoms or an alkenyl group having from 2 to 15 carbon atoms, preferably from 4 to 10 carbon atoms. Examples of the alkyl group include methyl, ethyl, n-propyl, isopropyl, n-butyl, isobutyl, tert-butyl, n-pentyl, n-hexyl, n-heptyl, n-octyl, n-nonyl and n-decyl. Examples of the alkenyl group include ethenyl, propenyl, butenyl, pentenyl, hexenyl, heptenyl, octenyl, nonenyl and decenyl. The alkyl groups are preferred.

The indolylacetic acid derivatives of general formula (I) can be prepared by various processes. Typical example thereof is described below.

Process (1)

A compound of general formula (II):

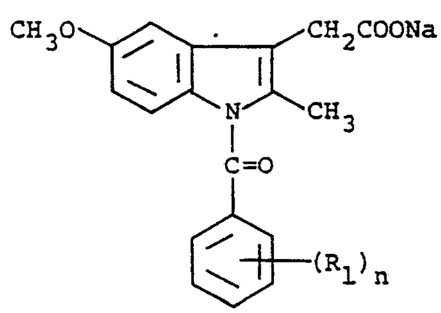

(II)

wherein $R_1$ and n are the same as defined in general formula (I) is reacted with an ester compound of general formula (III) or (IV)

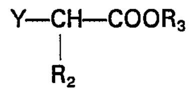 (III)

wherein $R_2$ and $R_3$ are the same as defined in general formula (I) and Y represents a halogen atom.

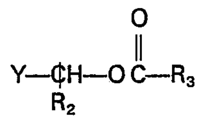 (IV)

wherein Y, $R_2$ and $R_3$ are the same as defined in general formula (III).

In accordance with this process, when the compound of general formula (III) is used, compounds of general formula (I) wherein X = —COO— can be obtained. On the other hand, when the compound of general formula (IV) is used, compounds of general formula (I) wherein

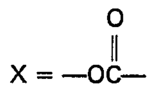

can be obtained.

In general formula (III) or (IV), Y is a halogen atom such as chlorine, bromine and iodine, and preferably an iodine atom.

The above reaction is carried out in the presence of a solvent. Examples of such solvents include methylene chloride and chloroform. The solvent is used preferably in an amount of from 1 to 10 ml per mmol of the compound of general formula (II).

The molar proportion of the compound of general formula (III) or (IV) to the compound of general formula (II) is preferably from 1.2:1 to 1.5:1.

It is preferred that the reaction be carried out in the presence of a catalyst (e.g., quaternary ammonium salts such as $(CH_3)_4NBr$, $(C_3H_7)_4NBr$, and $(C_4H_9)_4NHSO_4$) in an amount of from 1.1 to 1.5 mols per mol of the compound of general formula (II). In this case, the reaction can proceed in an anhydrous condition.

Usually, the reaction proceeds at a temperature of from 0 to 25°C for from 2 to 4 hours.

The compounds of general formula (II) can be prepared according to Process 2 below. Indomethacin of formula below

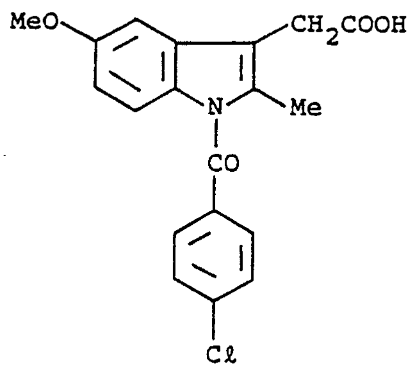

is commercially available.

The compounds of general formula (III) can be prepared from an α-halogenoalkanoic acid and an alkyl or alkenyl alcohol according to a conventional method.

On the other hand, the compounds of general formula (IV) can be prepared from a 1-halogen alkyl alcohol and an alkanoic or alkenoic acid in a conventional manner.

Process (2)

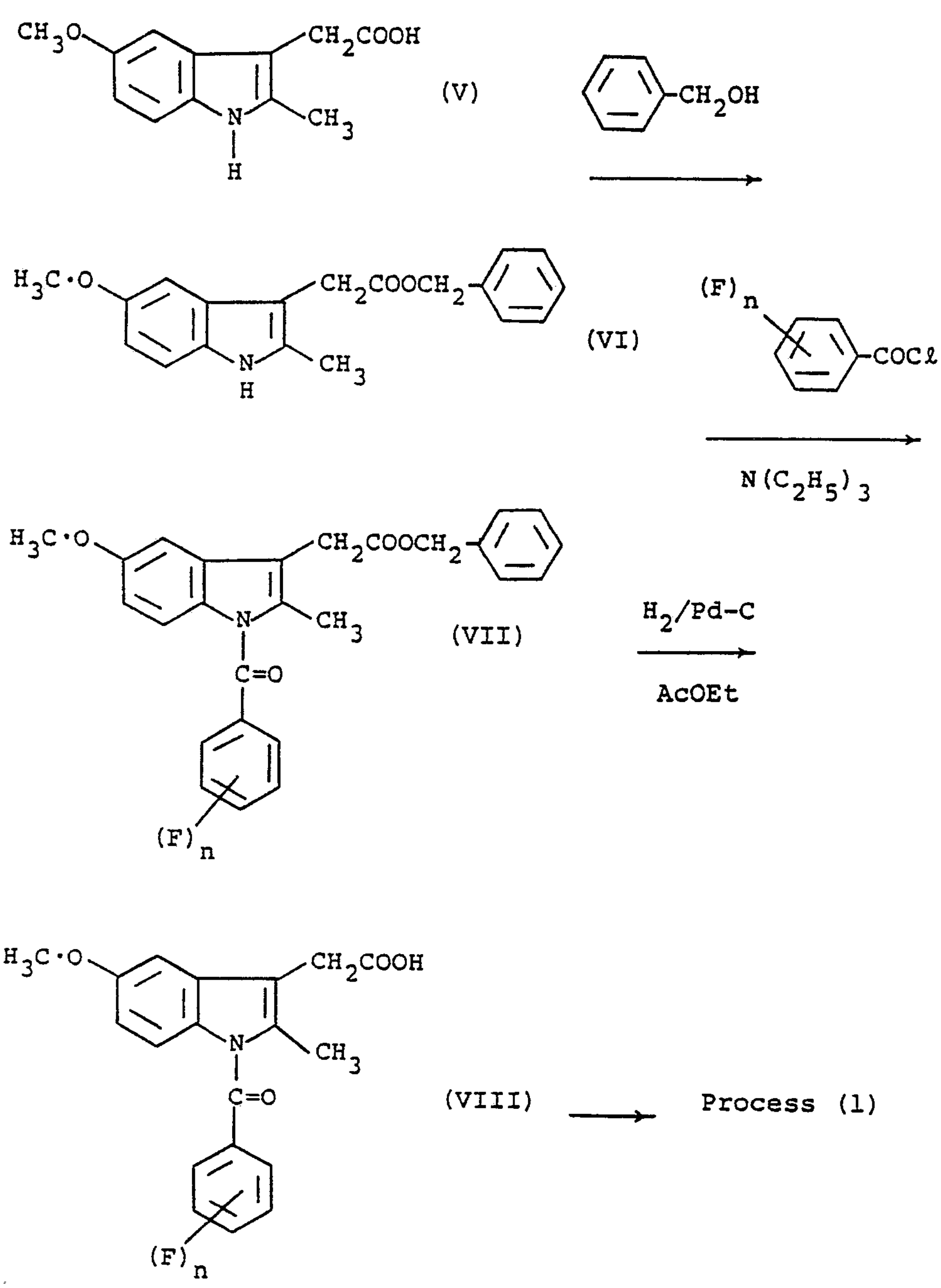


In the above formulae, n is an integer of 1 to 3.

A compound of formula (V), which is commercially available, can be converted into a benzyl ester of formula (VI) to protect its carboxylic acid moiety. Generally, the compound of formula (V) and benzyl alcohol (1:1.1 to 1:1.5 by mol) are dissolved in an organic solvent such as benzene in a concentration of from 0.1 to 1 mmol/ml heated under reflux in the presence of p-toluenesulfonic acid as a catalyst for from 3 to 5 hours to form an ester of formula (VI).

This ester is then reacted with fluorobenzoic acid chloride (1.1 to 1.5 mols of benzyl alcohol per mol of the compound of formula (VI)) in an organic solvent such as dimethylethane in the presence of triethylamine (0.1 to 1 mol of triethylamine per mmol of the compound of formula (VI)) under ice cooling for from 2 to 4 hours. After addition of pyridine the reaction is continued fro additional 2 days to obtain an amide of formula (VII).

The benzyl ester moiety of the amide of formula (VII) is removed by, for example, catalytic hydrogenation decomposition using 10% palladium-carbon in an amount of from 5 to 100 mg per mmol of the compound of formula (VII) as a catalyst and supplying hydrogen gas (from 20 to 40 ml per mmol of the

compound of formula (VII) for from 2 to 4 hours to obtain a compound of formula (VIII). The reaction conditions are not restricted particularly, and the reaction proceeds at normal temperature (0 to 25°C) and normal pressure.

After converting the carboxylic acid into sodium salt (—COONa), a compound of the formula (IX):

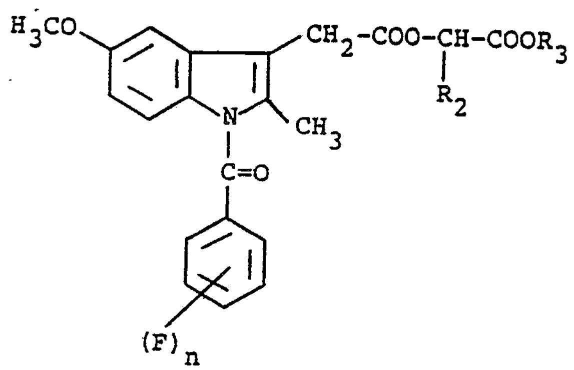

(IX)

wherein $R_2$, $R_3$ and n are the same as defined above, prepared according to Process (1) above.

The indolylacetic acid derivatives of general formula (I) are isolated and purified by known techniques such as solvent extraction, recrystallization, and chromatographic treatment.

The thus-obtained indolylacetic acid derivative was tested for its effect of inhibiting a carrageenan-induced edema in rats. The inhibitory rate of the derivative, when administered intravenously in a dosage of 0.3 mg/kg, was 30 to 60%. This demonstrates that the compound of the present invention is significantly effective in inhibiting an edema.

The indolylacetic acid derivative of general formula (I) is administered, orally or parenterally, in the form of preparations for medical treatment comprising the derivative and suitable and commonly used pharmaceutically acceptable carriers. The medical preparation can take a usual form such as a tablet, a capsule, a bulky powder, a suppository, and an injection. In oral administration, for example, the indolylacetic acid derivative of general formula (I) is usually administered once or several times a day in a dosage of from 10 to 300 mg, although the dosage may vary depending on the age, weight, symptom, and response to treatment of patients.

The indolylacetic acid derivative of general formula (I) exhibits a high solubility in lipid (1—10 mg/g of vegetable oil) and can be converted into a fat emulsion in any proportion by a known technique as described in, for example, U.S. Patent 4,280,996. Particularly when $R_1$ is fluorinated, the solubility in lipid is greatly high. As the number of the fluorine substituents is increased, the solubility in lipid is more increased. Such fat emulsions can be prepared by known techniques. Preferred emulsions are prepared by using vegetable oils such as soybean oil, cotton seed oil, rubber oil, safflower oil, and corn oil. Soybean oil is preferred.

The present invention is described in greater detail with reference to the following examples.

## Example 1

340mg (1 mmol) of tetrabutylammonium hydrogensulfate and 360 mg (1 mmol) of indomethacin were added in this order to 2 ml (2 mmol) of 1 N sodium hydroxide while cooling with ice and thoroughly dissolved therein. To the resulting solution was added 3 ml of dichloromethane, and the mixture was then well stirred. A dichloromethane layer was separated, and 3 ml of dichloromethane was further added to perform extraction. The dichloromethane extract was dried over anhydrous sodium sulfate for 30 minutes. After the sodium sulfate was separated by filtration, 1.2 mmol of butyl acetate iodide was added and reacted at room temperature for 3 hours. After the reaction was completed, 1 ml of water was added and the resulting mixture was well stirred. Thereafter, an organic layer was separated and dried over anhydrous sodium sulfate. The solvent was separated by filtration (a rotary evaporator, room temperature), thereby yielding a crude product. This crude product was purified by silica gel column chromatography (silica gel) to obtain, as white crystals, 499 mg of butoxycarbonylmethyl indomethacin ester, m.p. 74—75°C. The yield was 87%.

IR (KBr): 2950, 1755, 1730, 1670, 1620, 1600, 1590, 1480, 1360, 1220, 1200, 1140, 1130, 1090, 1070, 790, 755 $cm^{-1}$.

NMR ($CDCl_3$): 0.7—1.8 (m, 7H), 2.35 (s, 3H), 3.75 (s, 2H), 3.80 (s, 3H), 4.1 (t, 2H, J=7Hz), 4.6 (s, 2H), 6.5—7.7 (7H).

## Example 2

Hexyloxycarbonymethyl indomethacin ester was prepared in the same manner as in Example 1 except that hexyl iodoacetate was used as an alkylating agent. Yield: 91% m.p.: 61—62°C.

NMR ($CDCl_3$): 0.7—1.8 (m, 11H), 2.35 (s, 3H), 3.75 (s, 2H), 3.80 (s, 3H), 4.1 (t, 2H), 4.6 (s, 2H), 6.6—7.7 (m, 7H).

Example 3

Pivaloyloxymethyl indomethacin ester was prepared in the same manner as in Example 1 except that iodomethyl pivalate was used as an alkylating agent. Yield: 94%; yellow oily product.

IR (liquid membrane): 2950, 1750, 1680, 1600, 1460, 1360, 1320, 1220, 1100, 990, 840, 760 $cm^{-1}$.
NMR ($CDCl_3$): 1.1 (s, 9H), 2.35 (s, 3H), 3.65 (s, 2H), 3.80 (s, 3H), 5.7 (s, 2H), 6.6—7.7 (m, 7H).

Example 4

2 g (9.1 mmol) of 2-methyl-5-methoxyindolylacetic acid and 1.08 g (10 mmol) of benzyl alcohol were dissolved in 30 ml of benzene, and 30 mg of p-toluenesulfonic acid was added thereto. The resulting mixture was then refluxed for 4 hours using a water separator. After the reaction was completed, 40 ml of ethyl acetate was added, and an organic layer was washed with 30 ml of 5% sodium hydroxide, twice with 30 ml of water, and then with brine. After the layer was dried, the solvent was removed by evaporation, thereby yielding 2.96 g of benzyl 5-methoxy-2-methylindolyl-3-acetate. 1.35 g (4.4 mmol) of the above-obtained benzyl ester was dissolved in 7 ml of dichloromethane, and 0.63 ml of triethylamine was added thereto. The resulting mixture was cooled with ice. 2 ml of a solution of 714 mg (4.5 mmol) of p-fluorobenzoic acid chloride in 2 ml of dichloromethane was added dropwise thereto. After 3 hours, 1 ml of pyridine was added, and the resulting mixture was stirred for 2 days. After the reaction was completed, the reaction mixture was treated in the usual manner, thereby yielding a crude product. The crude product was eluted with a hexane/ethyl acetate (3:1) mixture over 60 g of silica gel to obtain benzyl 1-(p-fluorobenzoyl)-5-methoxy-2-methylindolyl-3-acetate (565 mg, 57%). 530 mg (1.2 mmol) of the above-obtained ester was dissolved in 5 ml of ethyl acetate and hydrogenated under atmospheric pressure by the use of 10% palladium-carbon. 30 ml of hydrogen was consumed in 4 hours. After the reaction was completed, the catalyst was removed by filtration, and the filtrate was evaporated, thereby yielding an oily crude product. This crude product was recrystallized from ether to yield 1-(p-fluorobenzoyl)-5-methoxy-2-methylindolyl-3-acetic acid (385 mg, 94%). In the same manner as in Example 1 except that the above acetic acid was used in place of indomethacin, butoxycarbonylmethyl 1 - (p - fluorobenzoyl) - 5 - methoxy - 2 - methylindolyl - 3 - acetate was obtained. Yield: 82%; m.p.: 61.0—62.5°C.

IR (KBr): 2950, 1740, 1730, 1665, 1600, 1505, 1470, 1440, 1370, 1320, 1280, 1235, 1210, 1160, 1075, 1030, 955, 915, 850, 790, 760, 610 $^{-1}$.
NMR ($CDCl_3$): 0.8—1.8 (m, 7H), 2.4 (s, 3H), 3.75 (s, 2H), 3.80 (s, 3H), 4.1 (t, 2H), 4.6 (s,2H), 6.5—7.8 (m, 7H).

In order to examine the solubility of the above-obtained substance in soybean oil, 200 mg of the substance was dissolved in 1 ml of soybean oil while heating at 70°C and, thereafter, the resulting solution was allowed to stand at 25°C for 2 days and nights. Precipitation of crystals was not observed at all, it was stable.

Preparation Example 1

24.0 g of purified yolk phospholipid and 400 mg of butoxycarbonylmethyl indomethacin ester were added to 200 g of purified soybean oil and dissolved therein by heating at 50 to 80°C. The purified yolk phospholipid was uniformly dispersed using a homogenizer.

50 g of glycerol was added to about 1.4 l of distilled water and dissolved therein by heating to prepare a uniform solution.

The above-prepared dispersion was added to the aqueous glycerol solution, and distilled water was added thereto so that the total amount was 2 l. Thereafter the resulting mixture was roughly emulsified.

The emulsion was further emulsified in a high pressure injection type emulsifier for about 1 to 2 hours under such pressure conditions that the initial pressure was 120 kg/$cm^2$ and the total pressure was 560 kg/$cm^2$. During this emulsification process, the temperature of the emulsion was maintained at 65 to 75°C. This resulted in the formation of a fat emulsion containing the indomethacin derivative. This emulsion was homogeneous, and the average particle diameter was 0.2 to 0.4 μ and particles having a diameter of more than 1 μ were not contained.

Preparation Example 2

An emulsion was prepared in the same manner as in Preparation Example 1 except that butoxycarbonylmethyl indomethacin ester was replaced by pivaloyloxymethyl indomethacin ester.

Preparation Example 3

An emulsion was prepared in the same manner as in Preparation Example 1 except that butoxycarbonylmethyl indomethacin ester was replaced by 1 - (p - fluorobenzoyl) - 5 - methoxy - 2 - methylindolyl - 3 - acetic acid butoxycarbonylmethyl ester.

Preparation Example 4

| | |
|---|---|
| Butoxycarbonyl Indomethacin Ester | 50 mg |
| Magnesium Stearate | 50 mg |
| Lactose | 50 mg |

The above ingredients were compounds to produce tablets each weighing 150 mg.

EXPERIMENT

Anti-inflammatory Action

Wister male rats were divided into groups of 6 rats each. 0.1 ml of a 1% solution of carrageenan in physiological saline was injected subcutaneously in the heel of a right hind leg for each rat to develop a leg edema. One hour after the injection of carrageenan, a drug to be tested was injected in a dosage of 0.3 mg/kg by the tail venous route. Thereafter the leg volume was measured with suitable time intervals.

The fat emulsions prepared in Preparation Examples 1 to 4 were tested. As a control drug, physiological saline was used.

The degree of the anti-inflammatory action was indicated as an inhibitory ratio in connection with the leg volume of physiological saline-administered groups 3 hours after the injection of carrageenan.

The results obtained are shown in Table 1.

TABLE 1

| Example No. | $R_1$ | $R_2$ | $R_3$ | X | Anti-inflammatory Action Edema Inhibition Ratio (%) |
|---|---|---|---|---|---|
| 1 | Cl | h | $—C_4H_9$ | —COO— | 60 |
| 2 | Cl | H | $—C_6H_{13}$ | —COO— | 62 |
| | Cl | H | $—C_8H_{17}$ | —COO— | 52 |
| | Cl | H | $—C_{10}H_{21}$ | —COO— | 49 |
| 3 | Cl | H | $—C_4H_9$ (t-Butyl) | —OCO— | 58 |
| 4 | F | H | $—C_4H_9$ | —COO— | 68 |
| | F | H | $—C_6H_{13}$ | —COO— | 66 |
| | F | H | $—C_8H_{17}$ | —COO— | 56 |
| | F | H | $—C_{10}H_{21}$ | —COO— | 40 |
| | Di-F | H | $—C_8H_{17}$ | —COO— | 60 |
| | Tri-F | H | $—C_{10}H_{21}$ | —COO— | 64 |

Further, in animal tests oral administration of 0.3 mg/kg of the indolylacetic acid derivatives of general formula (I) results in the formation of ulcer in, e.g., stomach, while the derivatives in the form of fat emulsions when administered intravenously do not lead to the formation of stomach ulcer.

While the invention has been described in detail and with reference to specific embodiments thereof, it will be apparent to one skilled in the art that various changes and modifications can be made therein without departing from the spirit and scope thereof.

**Claims**

1. An indolylacetic acid derivative represented by general formula I:

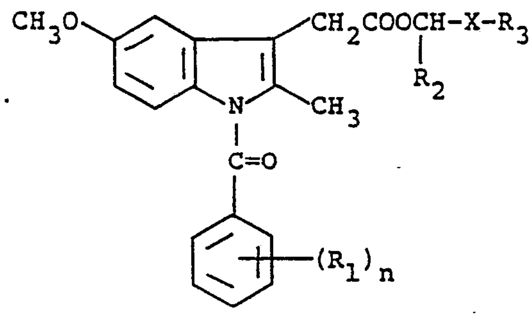

(I)

wherein $R_1$ represents a halogen atom, $R_2$ represents a hydrogen atom or an alkyl group, having from 1 to 5 carbon atoms, X represents

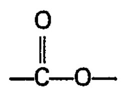 or 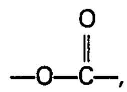,

$R_3$ represents an alkyl group having from 1 to 15 carbon atoms or an alkenyl group having from 2 to 15 carbon atoms, and n is an integer of from 1 to 3.

2. The derivative as claimed in Claim 1, wherein $R_1$ is a fluorine atom and n is 1.

3. The derivative as claimed in Claim 1, wherein $R_1$ is a fluorine atom and n is 2.

4. The derivative as claimed in Claim 1, wherein $R_1$ is a fluorine atom and n is 3.

5. The derivative as claimed in Claim 1, 2 or 3, wherein $R_2$ is a hydrogen atom.

6. The derivative as claimed in Claim 1, 2, 3 or 5, wherein X is

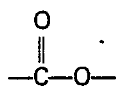

7. The derivative as claimed in any one of Claims 1 to 6, wherein $R_3$ is an alkyl group having from 4 to 10 carbon atoms or an alkenyl group having from 4 to 10 carbon atoms.

8. A medical preparation containing an indolylacetic acid derivative of the general formula I as shown below as an active ingredient:

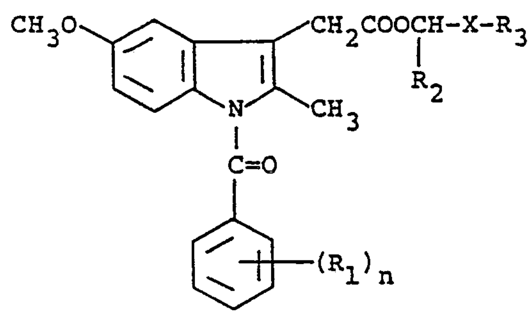

(I)

wherein $R_1$ represents a halogen atom, $R_2$ represents a hydrogen atom or an alkyl group, having from 1 to 5 carbon atoms, X represents

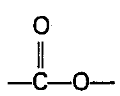 or 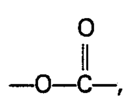,

$R_3$ represents an alkyl group having from 1 to 15 carbon atoms or an alkenyl group having from 2 to 15 carbon atoms, and n is an integer of from 1 to 3.

**Patentansprüche**

1. Ein Indolylessigsäurederivat der allgemeinen Formel I

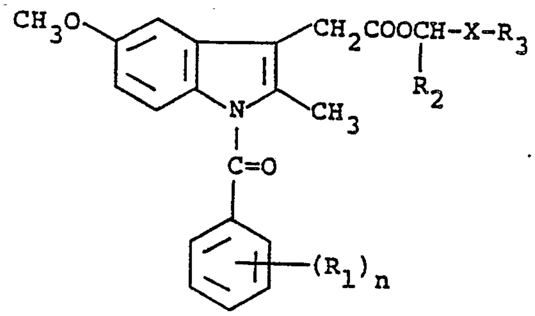

(I)

worin bedeuten:
$R_1$ ein Halogenatom,
$R_2$ ein Wasserstoffatom oder eine Alkylgruppe mit 1 bis 5 Kohlenstoffatomen,
X

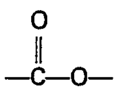 oder 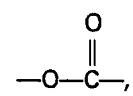,

$R_3$ ein Alkylgruppe mit 1 bis 15 Kohlenstoffatomen oder eine Alkenylgruppe mit 2 bis 15 Kohlenstoffatomen, und
n eine ganze Zahl von 1 bis 3.

2. Derivat gemäß Anspruch 1, bei dem $R_1$ ein Fluoratom ist und n 1 ist.
3. Derivat gemäß Anspruch 1, bei dem $R_1$ ein Fluoratom ist und n 2 ist.
4. Derivat gemäß Anspruch 1, bei dem $R_1$ ein Fluoratom ist und n 3 ist.
5. Derivat gemäß Anspruch 1, 2 oder 3, bei dem $R_2$ ein Wasserstoffatom ist.
6. Derivat gemäß Anspruch 1, 2, 3 oder 5, bei dem X

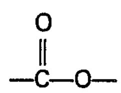

ist.

7. Derivat gemäß einem der Ansprüche 1 bis 6, bei dem $R_3$ ein Alkylgruppe mit 4 bis 10 Kohlenstoffatomen oder eine Alkenylgruppe mit 4 bis 10 Kohlenstoffatomen ist.
8. Medizinische Zubereitung, enthaltend ein Indolylessigsäurederivat der allgemeinen Formel I wie nachfolgend gezeigt als aktiven Bestandteil:

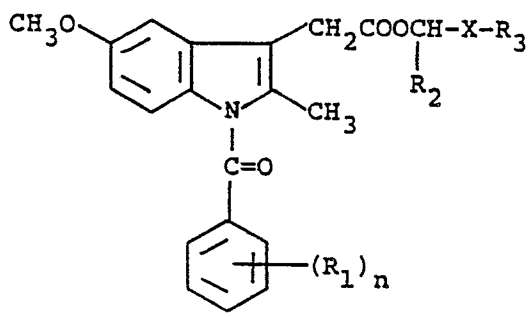

(I)

worin bedeuten:
$R_1$ ein Halogenatom
$R_2$ ein Wasserstoffatom oder eine Alkylgruppe mit 1 bis 5 Kohlenstoffatomen
X

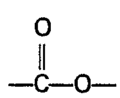 oder 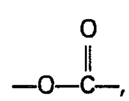,

$R_3$ eine Alkylgruppe mit 1 bis 15 Kohlenstoffatomen oder eine Alkenylgruppe mit 2 bis 15 Kohlenstoffatomen und
n eine ganze Zahl von 1 bis 3.

**Revendications**

1. Dérivé d'acide indolylacétique représenté par la formule générale:

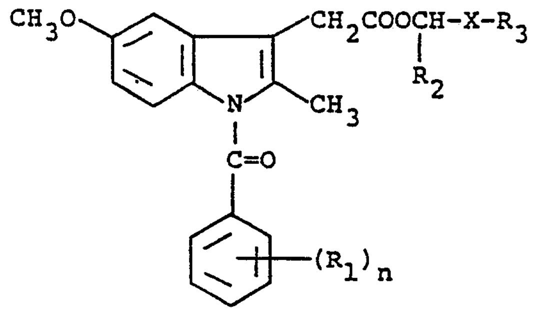

(I)

dans laquelle $R_1$ représente un atome d'halogène, $R_2$ représente un atome d'hydrogène ou un groupe alkyle en $C_1$—$C_5$, X représente

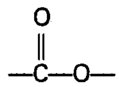 ou 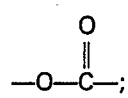

$R_3$ représente un groupe alkyle en $C_1$—$C_{15}$ ou alcényle en $C_2$—$C_{15}$ et n est un entier de 1 à 1.

2. Le dérivé selon la revendication 1, dans lequel $R_1$ est un atome de fluor et n est égal à 1.

3. Le dérivé selon la revendication 1, dans lequel $R_1$ est un atome de fluor et n est égal à 2.

4. Le dérivé selon la revendication 1, dans lequel $R_1$ est un atome de fluor et n est égal à 3.

5. Le dérivé selon la revendication 1, 2 ou 3, dans lequel $R_2$ est un atome d'hydrogène.

6. Dérivé selon la revendication 1, 2, 3 ou 5, dans lequel X est

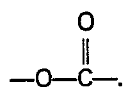

7. Le dérivé selon l'une quelconque des revendications 1 à 6, dans lequel $R_3$ est un groupe alkyle en $C_4$—$C_{10}$ ou alcényle en $C_4$—$C_{10}$.

8. Préparation médicale contenant comme ingrédient actif un dérivé d'acide indolylacétique de formule générale ci-dessous:

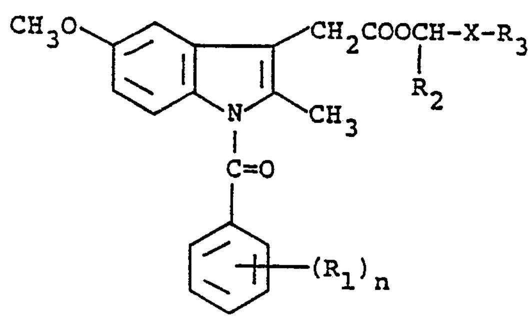

(I)

dans laquelle $R_1$ représente un atome d'halogène, $R_2$ représente un atome d'hydrogène ou un groupe alkyle en $C_1$—$C_5$, X représente

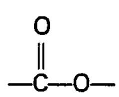 ou 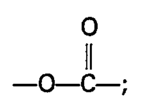

$R_3$ représente un groupe alkyle en $C_1$—$C_{15}$ ou alcényle en $C_2$—$C_{15}$ et n est un entier de 1 à 3.